# EUROPÄISCHE PATENTSCHRIFT

(11) **EP 2 723 482 B1**
(45) Veröffentlichungstag und Bekanntmachung des Hinweises auf die Patenterteilung: **29.06.2016**
(21) Anmeldenummer: 12724854.0
(22) Anmeldetag: 22.05.2012
(51) Int. Cl.: B01F 11/00, B01F 13/00, B01F 15/00, B01F 15/02, A61B 17/88

(54) **VORRICHTUNG ZUM BLASENARMEN MISCHEN UND AUSTRAGEN EINES PRODUKTS**
DEVICE FOR LOW-BUBBLE MIXING AND DISCHARGE OF A PRODUCT
DISPOSITIF DESTINÉ À MÉLANGER ET APPLIQUER UN PRODUIT AYANT PEU DE BULLES

(30) Priorität: 22.06.2011 CH 10562011
(43) Veröffentlichungstag der Anmeldung: 30.04.2014
(73) Patentinhaber: Medmix Systems AG, 6343 Rotkreuz (CH)
(72) Erfinder: GRETER, Andy, CH-6343 Rotkreuz (CH)
(74) Vertreter: Rutz, Andrea
(86) Internationale Anmeldenummer: PCT/CH2012/000116
(87) Internationale Veröffentlichungsnummer: WO 2012/174670

(56) Entgegenhaltungen:
- EP-B1- 1 359 872
- GB-A- 2 338 428
- US-A- 2 875 761
- US-A1- 2009 281 549

## Beschreibung

### TECHNISCHES GEBIET

Die vorliegende Erfindung betrifft eine Vorrichtung, um ein Produkt zu durchmischen und anschliessend aus der Vorrichtung auszutragen. Bei dem Produkt kann es sich insbesondere um ein Produkt aus zwei Komponenten, zum Beispiel aus einer pulverförmigen und einer flüssigen Komponente, handeln. Insbesondere eignet sich die Vorrichtung zur Herstellung und zum Austragen von Knochenzement.

### STAND DER TECHNIK

Aus der WO 2009/105905 ist eine spritzenartige Vorrichtung zum Mischen und Austragen eines Produkts aus zwei oder mehr Komponenten bekannt. Die Vorrichtung weist einen Spritzenbehälter mit einem darin verschiebbaren Kolben auf. Der Spritzenbehälter und der Kolben begrenzen gemeinsam ein Reservoir für die Komponenten. Eine Betätigungsstange ist durch den Kolben hindurchgeführt. Am distalen Ende der Betätigungsstange befindet sich innerhalb des Reservoirs ein Mischelement, das mit Hilfe der Betätigungsstange im Reservoir hin und her bewegt werden kann, um die Komponenten zu vermischen. Am proximalen Ende der Betätigungsstange ist schwenkbar ein Kopplungselement in Form einer seitlich offenen, hülsenartigen Kolbenstange angebracht. Das Kopplungselement lässt sich derart über die Betätigungsstange schwenken, dass es den Kolben am Mischelement fixiert und dadurch das Mischelement mit dem Kolben koppelt. Dadurch lässt sich der Kolben gemeinsam mit dem Mischelement in die distale Richtung vorschieben, um das fertige Produkt aus dem Reservoir auszutragen.

Bei einer derartigen Vorrichtung besteht die Möglichkeit, dass sich beim Mischen der Komponenten auch noch Luft und/oder andere Gase, z.B. Gase, die durch eine chemische Reaktion der Komponenten entstehen können, im Reservoir befinden. Beim Vermischen der Komponenten kann dies zur Blasenbildung im auszutragenden Produkt führen. Dies ist unerwünscht.

Um eine Blasenbildung zu vermeiden, sind aus dem Stand der Technik diverse Vorrichtungen bekannt, bei denen der Mischvorgang erfolgt, während an das Reservoir an Vakuum appliziert wird. Ein Beispiel für eine solche Vorrichtung ist in der US 2008/0144428 angegeben. Derartige Vorrichtungen erfordern jedoch eine Vakuumpumpe. Ausserdem ist die Handhabung einer solchen Vorrichtung wegen des erforderlichen Vakuumsschlauchs erschwert.

Die EP 1 359 872 offenbart eine Misch- und Austragvorrichtung für einen Knochenzement, bei welcher in einem Mischbehälter sowohl ein federbelasteter Kolben als auch ein Mischelement angeordnet sind.

### DARSTELLUNG DER ERFINDUNG

In einem ersten Aspekt der vorliegenden Erfindung ist es daher eine Aufgabe, eine Misch- und Austragvorrichtung anzugeben, bei der die Blasenbildung auch ohne die Applikation eines Vakuums vermindert ist. Diese Aufgabe wird durch eine Vorrichtung mit den Merkmalen des Anspruchs 1 gelöst. Weitere Ausführungsformen sind in den abhängigen Ansprüchen angegeben.

Die Erfindung stellt also eine Vorrichtung zum Mischen und Austragen eines Produkts zur Verfügung, welche aufweist:
ein Gehäuse, welches ein Reservoir für das Produkt begrenzt;
ein Mischelement, welches im Reservoir bewegbar ist, um ein darin aufgenommenes Produkt zu durchmischen; und
einen Kolben, welcher das Reservoir zu einem proximalen Ende hin begrenzt und welcher relativ zum Gehäuse in eine distale Richtung verschiebbar ist, um das Produkt in die distale Richtung aus dem Reservoir auszutragen.

Erfindungsgemäss weist die Vorrichtung ein Federelement auf, das dazu ausgebildet ist, den Kolben gegenüber dem Gehäuse in die distale Richtung mit einer Federkraft zu belasten.

Indem ein solches Federelement vorgesehen wird, kann sichergestellt werden, dass der Kolben schon vor dem Beginn des Mischvorgangs satt an dem zu durchmischendem Produkt bzw. den zu vermischenden Komponenten anliegt und sich im Wesentlichen keine Luft mehr in dem Reservoir befindet. Dadurch wird die Blasenbildung während des Mischens vermindert. Zudem ermöglicht es diese Vorrichtung, dass andere Gase, die möglicherweise im Zuge von chemischen Reaktionen im zu vermischenden Produkt entstehen, aus dem Reservoir entfernt werden.

Um das Befüllen der Vorrichtung zu erleichtern, ist vorzugsweise eine Fixiereinrichtung vorgesehen, um den Kolben in einer Haltestellung relativ zum Gehäuse zu fixieren, wobei in dieser Haltestellung das Federelement vorgespannt ist. Auf diese Weise wird es insbesondere möglich, das Reservoir zu befüllen, während der Kolben in der Haltestellung fixiert ist. Wenn der Kolben anschliessend aus der Haltestellung freigegeben wird, drückt das Federelement den Kolben in die distale Richtung nach vorne und presst so die nach dem Befüllen möglicherweise noch im Reservoir befindliche Luft aus dem Reservoir hinaus. Mögliche konkrete Ausgestaltungen der Fixiereinrichtung werden nachfolgend noch näher beschrieben.

Um bei der durch das Federelement bewirkten Bewegung des Kolbens in die distale Richtung einen Austritt von Luft und anderen Gasen aus dem Reservoir zu ermöglichen, weist die Vorrichtung mindestens eine Entlüftungsöffnung auf. Diese ist vorzugsweise derart ausgebildet, dass sie einen Austritt von Gasen erlaubt, während sie einen Austritt von pulver- oder pastenförmigen Feststoffen verhindert. Dazu kann die Entlüftungsöffnung z.B. einen genügend kleinen Querschnitt aufweisen, der den Durchtritt von Partikeln einer vorgesehenen pulverförmigen Komponente verhindert, oder in der Entlüftungsöffnung kann z.B. ein Filter eingesetzt sein. Eine solche Entlüftungsöffnung ermöglicht es, dass der Kolben unter der Wirkung des Federelements das im Reservoir befindliche Gas aus dem Reservoir hinauspresst, ohne dass dabei auch das zu vermischende Produkt aus dem Reservoir hinausgepresst wird. Die Entlüftungsöffnung kann auch zusätzlich zum Zuführen und Abführen eines Gases für eine Sterilisation verwendet werden, z.B. von Ethylenoxid. In diesem Fall ist die Verwendung eines Filters in der Entlüftungsöffnung besonders bevorzugt.

Vorzugsweise ist die Entlüftungsöffnung am Kolben ausgebildet. Dazu ist es denkbar, die Entlüftungsöffnung als Durchgangsöffnung im Körper des Kolbens selbst vorzusehen, es ist aber auch denkbar, eine solche Öffnung zwischen einem äusseren Randbereich des Kolbens und einer umlaufenden Seitenwand des Behälters vorzusehen. Bevorzugt ist die Entlüftungsöffnung jedoch zwischen dem Kolben und dem Betätigungselement für das Mischelement ausgebildet, wobei das Betätigungselement den Kolben durchsetzt.

In einer vorteilhaften Ausgestaltung ist die Entlüftungsöffnung durch das Mischelement und/oder das Betätigungselement verschliessbar. Diesbezüglich wird auf die Patentanmeldung PCT/CH 2011/00135 vom 7. Juni 2011 verwiesen, deren Inhalt durch Verweis vollständig hierin aufgenommen wird. Das Mischelement beziehungsweise das Betätigungselement wirkt in diesem Fall also als Ventil für die Entlüftungsöffnung. Ein Ventil für die Entlüftungsöffnung kann aber auch auf eine andere Weise ausgebildet sein. Insbesondere kann es sich dabei auch um ein Einwegventil handeln, welches zwar das Entweichen von Gasen aus dem Reservoir erlaubt, aber ein Eintritt von Gasen in das Reservoir verhindert. Indem die Entlüftungsöffnung durch ein Ventil verschliessbar ist, wird es insbesondere möglich, den Kolben im Gehäuse in die proximale Richtung zurückzuziehen und so einen Unterdruck im Reservoir zu erzeugen, ohne dass dabei durch die Entlüftungsöffnung Luft angesaugt wird. Dies kann nützlich sein, um durch eine andere Öffnung des Reservoirs eine flüssige Komponente in das Reservoir einzusaugen. Eine Ventilwirkung ist auch von Vorteil, wenn vorgesehen ist, zum Zwecke der Sterilisation durch eine andere Öffnung dem Reservoir ein Gas zuzuführen, insbesondere durch einen geeigneten Filter hindurch. Das Ventil verhindert dann einen unkontrollierten Austritt des Gases.

Wie schon erwähnt, ist das Mischelement vorzugsweise mit einem Betätigungselement verbunden, welches den Kolben durchsetzt. Das Betätigungselement ragt dann in proximaler Richtung aus dem Reservoir heraus. Die Vorrichtung kann ausserdem ein Kopplungselement umfassen, welches in einen Zustand bringbar ist, in dem es das Betätigungselement bezüglich mindestens einer (der proximalen Richtung entgegengesetzten) distalen Richtung relativ zum Gehäuse fixiert. Dadurch können das Betätigungselement und/oder das damit verbundene Mischelement den Kolben in einer Haltestellung halten, in der das Federelement vorgespannt ist. In diesem Fall wird also der Kolben durch das Mischelement daran gehindert, sich aufgrund der Federkraft in die distale Richtung zu bewegen. In dieser Hinsicht bilden dann also das Mischelement, das Betätigungselement und das Kopplungselement gemeinsam die oben erwähnte Fixiereinrichtung für den Kolben.

Eine Fixiereinrichtung für den Kolben ist aber auch dann von Vorteil, wenn kein Federelement vorhanden ist. Dies kann von Vorteil sein, um den Kolben z.B. während einer Befüllung unter Vakuum oder während einer Sterilisation mit einem Gas und der anschliessenden Absaugung des Gases zu fixieren. Die Erfindung bezieht sich demgemäss gemäss einem zweiten Aspekt auch auf eine Vorrichtung zum Mischen und Austragen eines Produkts zur Verfügung, welche aufweist:
ein Gehäuse, welches ein Reservoir für das Produkt begrenzt;
einen Kolben, welcher das Reservoir zu einem proximalen Ende hin begrenzt und welcher relativ zum Gehäuse in eine distale Richtung verschiebbar ist, um das Produkt in die distale Richtung aus dem Reservoir auszutragen;
ein Mischelement, welches im Reservoir bewegbar ist, um ein darin aufgenommenes Produkt zu durchmischen;
eine Fixiereinrichtung, um den Kolben relativ zum Gehäuse zumindest bezüglich einer Bewegung in der distalen Richtung zu fixieren.

Wenn die Vorrichtung ein mit dem Mischelement verbundenes Betätigungselement aufweist, welches den Kolben durchsetzt und in proximaler Richtung aus dem Reservoir herausragt, um das Mischelement im Reservoir zu bewegen, kann die Fixiereinrichtung insbesondere das schon erwähnte Kopplungselement umfassen, welches in einen Zustand bringbar ist, in dem es das Betätigungselement bezüglich mindestens der distalen Richtung relativ zum Gehäuse fixiert, so dass das Betätigungselement und/oder das Mischelement den Kolben mindestens bezüglich der distalen Richtung in einer Haltestellung halten.

Bevorzugt ist das Kopplungselement ausserdem dazu ausgebildet, das Mischelement mit dem Kolben zu koppeln, indem es das Mischelement relativ zum Kolben fixiert. Dazu kann das Kopplungselement derart proximal vom Kolben am Betätigungselement anbringbar sein, dass der Kolben zwischen dem Kopplungselement und dem Mischelement gehalten ist. Dadurch wird es möglich, mit Hilfe des Kopplungselements den Kolben in die distale oder proximale Richtung zu bewegen, solange sich das Kopplungselement in einem Zustand befindet, in welchem es das Betätigungselement nur relativ zum Kolben, aber nicht relativ zum Gehäuse fixiert. Das Kopplungselement wirkt in diesem Fall also als Kolbenstange für den Kolben.

Insbesondere kann im Kolben eine zentrale Durchgangsöffnung vorgesehen sein, durch welche das Betätigungselement hindurchragt. In einer konkreten Ausgestaltung kann das Betätigungselement insbesondere eine Betätigungsstange bilden, und das Kopplungselement kann dann eine seitlich offene (d.h. lateral, quer zur Bewegungsrichtung des Kolbens offene) hohle Kolbenstange bilden, die von der Seite her (lateral) über das Betätigungselement bewegbar ist und die derart am Betätigungselement fixierbar ist, dass sie an einem proximalen Bereich des Kolbens anliegt. Eine solche Ausgestaltung des Betätigungselements und des Kopplungselements ist insbesondere in der schon erwähnten WO 2009/105905 offenbart, wobei es im vorliegenden Kontext jedoch bevorzugt ist, wenn das Kopplungselement nicht am Betätigungselement angelenkt ist, sondern vom Betätigungselement abnehmbar ist. Eine solche Ausgestaltung ist zudem auch in der schon erwähnten Anmeldung PCT/CH 2011/00135 vom 7. Juni 2011 offenbart.

Unabhängig von der konkreten Ausgestaltung des Betätigungselements und des Kopplungselements ist es bevorzugt, wenn die Vorrichtung eine lösbare Feststelleinrichtung aufweist, die in der Haltestellung das Kopplungselement relativ zum Gehäuse derart fixiert, dass die Feststelleinrichtung eine Bewegung des Kopplungselements zumindest in die distale Richtung blockiert, während die Feststelleinrichtung in einer Freigabestellung eine Bewegung des Kopplungselements gemeinsam mit dem Betätigungselement in die distale Richtung erlaubt. Die Feststelleinrichtung kann eine lösbare Rastverbindung zwischen dem Kopplungselement und dem Gehäuse bzw. einem gehäusefesten Element bilden und insbesondere eine Rastklinke am Kopplungselement umfassen. Diese greift dann in der Haltestellung vorzugsweise am Gehäuse oder einem gehäusefesten Element an, um eine Bewegung des Kopplungselements in die distale Richtung zu verhindern. Die Feststelleinrichtung lässt sich dann z.B. durch Eindrücken oder Entfernen der Rastklinke in die Freigabestellung bringen. Eine Vielzahl anderer Feststelleinrichtungen sind denkbar, wie nachstehend noch näher ausgeführt wird.

Das Betätigungselement kann mit einer Trennstelle versehen sein, die es ermöglicht, einen proximalen Teil des Betätigungselements gezielt zu entfernen. Dabei kann es sich z.B. um eine Sollbruchstelle handeln, um den proximalen Teil des Betätigungselements gezielt abzubrechen, oder um eine Schraubverbindung, um den proximalen Teil des Betätigungselements abzuschrauben. Dadurch wird es möglich, ein Auspresselement auf den Rest des Betätigungselements aufzuschieben, welches dazu dient, auf den Kolben eine Auspresskraft in die distale Richtung auszuüben, um das Produkt aus dem Reservoir auszutragen.

Insbesondere kann ein solches Auspresselement dazu ausgebildet sein, eine Gewindeverbindung mit einem gehäusefesten Gegenstück, z.B. einer gehäusefesten Gewindebuchse, einzugehen. Insbesondere kann das Gegenstück ein Innengewinde aufweisen, und das Auspresselement kann in diesem Fall ein Aussengewinde aufweisen, das mit dem Innengewinde im Eingriff bringbar ist. Das Auspresselement umfasst insbesondere bevorzugt eine hohle Gewindespindel mit einem Aussengewinde, wobei der Hohlraum geeignet ist, einen Teil des Betätigungselement aufzunehmen.

Das gehäusefeste Gegenstück ist vorzugsweise einstückig mit dem Gehäuse ausgebildet und in einem proximal vom Kolben gelegenen Bereich des Gehäuses angeordnet. Es ist aber auch denkbar, ein solches Gegenstück separat vom Gehäuse auszubilden und zum Beispiel auf das Gehäuse aufzuschnappen oder aufzuschrauben.

Gemäss einem weiteren Aspekt bezieht sich die vorliegende Erfindung auch auf eine Vorrichtung zum Mischen und Austragen eines Produkts, welches aufweist:
ein Gehäuse, welches ein Reservoir für das Produkt begrenzt;
einen Kolben, welcher das Reservoir zu einem proximalen Ende hin begrenzt und welcher relativ zum Gehäuse in eine distale Richtung verschiebbar ist, um das Produkt in eine distale Richtung aus dem Reservoir auszutragen; und
ein Auspresselement, um eine Auspresskraft in distaler Richtung auf den Kolben auszuüben, wobei das Auspresselement dazu ausgebildet ist, eine Gewindeverbindung mit einem proximal vom Kolben angeordneten, gehäusefesten Gegenstück einzugehen.

Dabei wird vorgeschlagen, das gehäusefeste Gegenstück unlösbar und bevorzugt einstückig mit dem Gehäuse auszubilden. Im Stand der Technik ist es zwar an sich bekannt, ein Auspresselement in Form einer Gewindespindel einzusetzen. Dieses wirkt jedoch im Stand der Technik normalerweise mit einem separat vom Gehäuse ausgebildeten Gegenstück zusammen, welches z.B. auf das Gehäuse aufschnappbar ist. Indem das gehäusefeste Gegenstück unlösbar oder sogar einstückig mit dem Gehäuse ausgebildet wird, werden sowohl die Fertigung als auch die Handhabung der Vorrichtung vereinfacht. Es ist nicht mehr nötig, das Gegenstück für das Auspresselement separat auf das Gehäuse aufzuschnappen, sondern das Auspresselement (das insbesondere in der Form einer Gewindespindel vorliegen kann) kann direkt in das Gehäuse eingeschraubt werden.

Auch eine solche Vorrichtung weist bevorzugt ein Mischelement auf, welches im Reservoir bewegbar ist, um ein darin aufgenommenes Produkt zu durchmischen. Das Mischelement ist wiederum bevorzugt mit einem Betätigungselement verbunden, welches den Kolben durchsetzt und in proximaler Richtung aus dem Reservoir herausragt, um das Mischelement im Reservoir zu bewegen. Auch in dieser allgemeineren Ausgestaltung weist das Betätigungselement vorzugsweise eine Sollbruchstelle auf, um ein proximalen Teil des Betätigungselements abzubrechen, sodass das Auspresselement auf den Rest des Betätigungselements aufschiebbar ist. Das Auspresselement kann jedoch auch auf das gesamte Betätigungselement aufschiebbar sein. Auch die weiteren Überlegungen, die vorstehen zum ersten Aspekt der Erfindung angestellt wurden, sind sinngemäss auf den zweiten Aspekt der Erfindung anwendbar.

Die erfindungsgemässe Vorrichtung kann insbesondere als ein Kit vorliegen, das einerseits das Gehäuse mit dem darin befindlichen Kolben und gegebenenfalls den weiteren genannten Elementen umfasst, und das andererseits das Auspresselement als separates Teil umfasst.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Fig. 1: eine Misch- und Austragvorrichtung gemäss einem bevorzugten Ausführungsbeispiel während des Füllens;
- Fig. 2: einen zentralen Längsschnitt durch die Vorrichtung der Fig. 1 im Bereich II der Fig. 1;
- Fig. 3: eine Ansicht der Vorrichtung der Fig. 1 nach dem Befüllen im verschlossenen Zustand;
- Fig. 4: einen zentralen Längsschnitt durch die Vorrichtung der Fig. 1 nach dem Entfernen des Kopplungselements;
- Fig. 5: einen zentralen Längsschnitt durch die Vorrichtung der Fig. 1 nach dem Mischvorgang, wobei das proximale Ende des Betätigungselements entfernt wurde;
- Fig. 6: einen zentralen Längsschnitt durch die Vorrichtung der Fig. 1 beim Austragen, mit eingeschraubtem Auspresselement;
- Fig. 7: einen zentralen Längsschnitt durch die Vorrichtung der Fig. 1 während des Befüllens mit einer anderen Befüllmethode; sowie
- Fig. 8: einen zentralen Längsschnitt durch die Vorrichtung der Fig. 1 während des Befüllens mit einer nochmals anderen Befüllmethode.

### BESCHREIBUNG BEVORZUGTER AUSFÜHRUNGSFORMEN

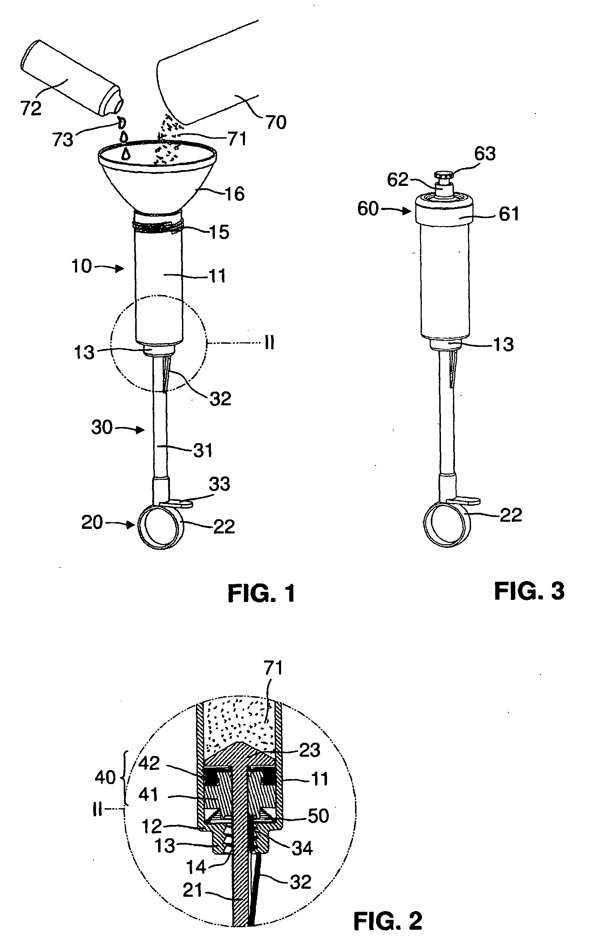

In den Figuren 1 bis 6 ist ein bevorzugtes Ausführungsbeispiel einer erfindungsgemässen Misch- und Austragvorrichtung illustriert. Die Vorrichtung weist ein Gehäuse 10 mit einer umlaufenden zylindrischen Seitenwand 11 auf. Das Gehäuse 10 ist an seinem distalen Ende (in der Fig. 1 ist dies das obere Ende) offen. Am proximalen Ende ist eine proximale Stirnwand 12 ausgebildet, an die sich in proximaler Richtung eine Gewindebuchse 13 mit einem Innengewinde 14 anschliesst. Die Stirnwand 12 und die Gewindebuchse 13 bilden im Bereich des Innengewindes 14 gemeinsam eine Durchgangsöffnung, durch welche eine nachstehend noch nähere beschriebene Betätigungsstange 21 für ein Mischelement 23 hindurchgeführt ist.

Im Gehäuse 10 ist ein Kolben 40 verschiebbar angeordnet. Der Kolben besteht aus einem Träger 41 mit einem daran angebrachten elastischen Dichtelement 42. Das Dichtelement liegt dabei dichtend an der umlaufenden Seitenwand 11 des Gehäuses 10 an. Der Kolben 40 begrenzt dadurch gemeinsam mit dem Gehäuse 10 ein Reservoir zur Aufnahme von Komponenten 71, 73.

Der Träger 41 und das Dichtelement 42 des Kolbens 40 weisen jeweils eine zentrale Bohrung auf, durch welche ein Betätigungselement in Form der schon erwähnten Betätigungsstange 21 hindurchgeführt ist. An ihrem distalen Ende ist die Betätigungsstange 21 mit einem Mischelement 23 verbunden. Das Mischelement weist dabei beispielsweise vier sich radial nach aussen erstreckenden Arme auf, die mit einem umlaufenden, konzentrisch zur Betätigungsstange 21 verlaufenden Ring oder vier separaten Ringsegmenten verbunden sein können. Das Mischelement kann aber auch auf eine andere, an sich aus dem Stand der Technik bekannte Weise ausgebildet sein, z. B. als eine Platte mit mehreren Durchgangsöffnungen. Das Mischelement 23 dient dazu, die in das Reservoir eingefüllten Komponenten 71, 73 zu durchmischen, indem es im Reservoir hin- und herbewegt und gegebenenfalls auch gedreht wird. Dazu ist die Betätigungsstange 21 an ihrem proximalen Ende mit einem Betätigungsring 22 verbunden. Die Betätigungsstange 21, der Betätigungsring 22 und das Mischelement 23 bilden also insgesamt eine Mischeinrichtung 20.

Zwischen der proximalen Stirnwand 12 des Gehäuses 10 und dem Träger 41 des Kolbens 40 ist eine spiralförmig gewickelte Schraubenfeder 50 aus einem geeigneten Material, z.B. aus Federstahl, angeordnet. In der Stellung der Figuren 1 bis 3 ist diese Feder komprimiert und übt dadurch auf den Kolben 40 eine Kraft in die distale Richtung aus. Um zu verhindern, dass sich die Feder 50 entspannt und dabei den Kolben 40 in die distale Richtung drückt, weist die Vorrichtung ein Kopplungselement 30 auf, welches eine seitlich offene Hülse 31 bildet. Diese Hülse 31 ist in den Figuren 1-3 seitlich über die Betätigungsstange 21 geschoben. Sie erstreckt sich mit ihrem distalen Ende 34 in die Durchgangsöffnung am proximalen Ende des Gehäuses 10 hinein und liegt dort auf dem Träger 41 des Kolbens 40 auf. Mit ihrem proximalen Ende liegt die Hülse 31 am Betätigungsring 22 an; in diesem Bereich ist an der Hülse 31 eine Grifflasche 33 ausgebildet. Insgesamt fixiert das Kopplungselement 30 auf diese Weise den Kolben 40 zwischen der Hülse 31 und dem Mischelement 23 und koppelt dadurch den Kolben 40 mit dem Mischelement 23 und der Betätigungsstange 21.

Nahe dem distalen Ende der Hülse 31 ist am Kopplungselement 30 eine Feststelleinrichtung in Form einer Sperrklinke oder Rastklinke 32 ausgebildet, die seitlich über die Hülse 31 vorsteht und einen Anschlag mit der gehäusefesten Gewindebuchse 13 bildet. Dadurch verhindert das Kopplungselement 30, dass sich die Betätigungsstange 21 und das damit verbundene Mischelement 23 in die distale Richtung ins Reservoir hineinbewegen lassen. Der Kolben 40 liegt am Mischelement 23 an und wird somit ebenfalls an einer Bewegung in die distale Richtung gehindert. Insgesamt ist dadurch der Kolben 40 relativ zum Gehäuse 10 in einer Haltstellung fixiert, in der das Federelement 50 vorgespannt ist. Das Kopplungselement 30 mit der Rastklinke 32, die Betätigungsstange 21 und das Mischelement 23 bilden hier also gemeinsam eine Fixiereinrichtung, um den Kolben 40 gehäusefest zu fixieren.

Indem der Kolben 40 in der Haltestellung der Fig. 2 gehalten ist, wird es möglich, die Vorrichtung auf die in der Fig. 1 gezeigte Weise zu befüllen. Dazu ist in das offene distale Ende des Gehäuses 10 ein Einfülltrichter 16 eingesetzt, mit dessen Hilfe z. B. aus einem Pulverbehälter 70 eine pulverförmige Komponente 71 und aus einem Flüssigkeitsbehälter 72 eine flüssige Komponente 73 in das Reservoir einfüllbar sind. Nach dem Einfüllen der Komponenten wird der Trichter 16 entfernt, und das Reservoir wird mit Hilfe eines Deckels 60 verschlossen. Der Deckel 60 weist dabei eine umlaufende Schürze 61 auf, an deren Innenseite ein Innengewinde ausgebildet ist, welches in ein Aussengewinde 15 auf der Aussenseite der Seitenwand 11 des Gehäuses 10 eingreift. Der Deckel weist ausserdem einen Austragnippel 62 auf, der eine Austragöffnung bildet, welche durch einen Verschluss 63 verschlossen ist. Dazu weist der Austragnippel 62 ein Innengewinde auf, in welches ein Aussengewinde des Verschlusses 63 eingreift. Nach dem Einfüllen der Komponenten 71, 73 und dem Verschliessen durch den Deckel 60 resultiert der Zustand der Fig. 3. Alternativ kann die Vorrichtung auch schon mit einer Komponente, vorzugsweise der pulverförmigen Komponente, vorbefüllt sein.

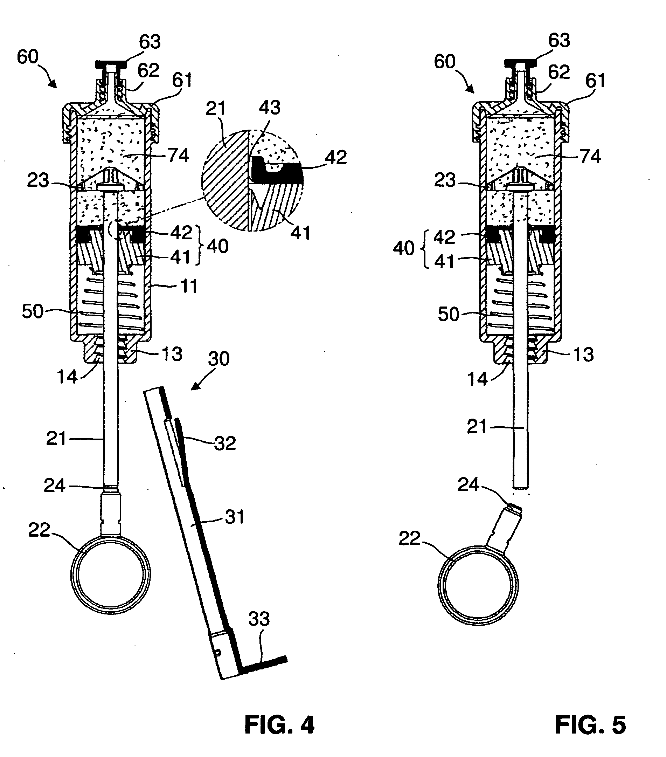

Um die eingefüllten Komponenten zu mischen, wird nun das Kopplungselement 30 von der Betätigungsstange 21 entfernt, wie dies in der Fig. 4 dargestellt ist. Dadurch kann sich nun die Betätigungsstange 21 mit dem Mischelement 23 gegenüber dem Kolben 40 in die distale Richtung bewegen. Da der Kolben 40 nun nicht mehr vom Mischelement 23 an einer Bewegung in die distale Richtung gehindert ist, kann sich nun auch der Kolben 40 unter der Wirkung der Feder 50 in die distale Richtung bewegen.

Zwischen dem Kolben 40 und der Betätigungsstange 21 ist mindestens eine Entlüftungsöffnung 43 ausgebildet (siehe den vergrösserten Ausschnitt in der Fig. 4; im vorliegenden Beispiel sind vorzugsweise drei oder vier Belüftungsöffnungen vorhanden, die gleichmässig in Umfangsrichtung verteilt angeordnet sind). Die Entlüftungsöffnung war während des Einfüllens durch das Mischelement 23 verschlossen. Nach der Freigabe des Mischelements 23 und des Kolbens 40 können nun Gase, die sich im Reservoir angesammelt haben, unter der Wirkung des federbelasteten Kolbens 40 durch die Entlüftungsöffnung 43 nach aussen austreten. Die Entlüftungsöffnung 43 ist dabei so klein gewählt, dass zumindest die pulverförmige Komponente 71 nicht durch diese Öffnung nach aussen gelangen kann. Eine flüssige Komponente 73 wiederum wird in der Regel von der pulverförmigen Komponente 71 so gut aufgenommen, dass sie gar nicht bis zur Entlüftungsöffnung 43 gelangt. Auf diese Weise gelangen in der Praxis nur Gase durch die Entlüftungsöffnung 43 nach aussen.

Im Ergebnis drückt also die Feder 50 den Kolben 40 so weit in die distale Richtung, dass Gase, insbesondere Luft, die sich im Reservoir befinden, weitgehend aus dem Reservoir ausgepresst werden. Die Wirkung der Feder 50 ist insofern ähnlich zu der Wirkung eines an das Reservoir angelegten Vakuums, indem auf diese Weise der grösste Teil der vorhandenen Gase aus dem Reservoir entfernt wird. Diese Wirkung wird jedoch auf eine sehr viel einfachere Weise als durch das Anlegen eines Vakuums erzielt.

Um das fertige Produkt 74 nach dem Mischen auszutragen, gibt es nun mehrere Möglichkeiten. Eine erste Möglichkeit besteht darin, die Betätigungsstange 21 wieder so weit in die proximale Richtung zurückzuziehen, bis das Mischelement 23 am Kolben 40 anstösst, und das Kopplungselement 30 wieder an der Betätigungsstange 21 anzubringen. Dazu kann es unter Umständen nötig sein, die Rastklinke 23 von Hand nach innen einzudrücken, so dass die Rastklinke 21 in die proximale Öffnung des Gehäuses eingeführt werden kann. Der Kolben 40 ist nun wieder durch das Kopplungselement 30 mit dem Mischelement 23 und der Betätigungsstange 21 gekoppelt. Nun kann der Verschluss 63 aus dem Austragnippel 62 entfernt werden, und das Produkt kann durch den Austragnippel 62 hindurch ausgetragen werden, indem die Betätigungsstange 21 in die distale Richtung nach vorne gedrückt wird.

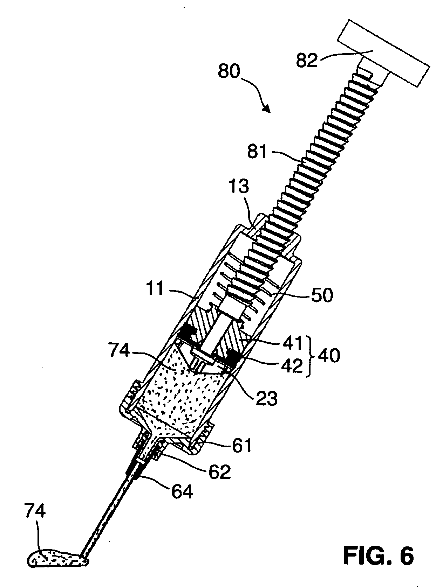

Häufig wird es sich bei dem fertigen Produkt 74 jedoch um eine sehr zähe Masse handeln, insbesondere wenn es sich bei dem Produkt 74 z.B. um einen Knochenzement handelt. In diesem Fall können für das Austragen grössere Kräfte erforderlich sein, als sie allein durch manuellen Druck auf die Betätigungsstange 21 aufgebracht werden können. Für diese Fälle steht eine zweite Möglichkeit zum Austragen des fertigen Produkts 74 zur Verfügung. Hierzu weist die Betätigungsstange 21 einen Schwächungsbereich 24 auf, an dem der proximale Bereich der Betätigungsstange mit dem Betätigungsring 22 entfernt, insbesondere abgebrochen werden kann. Dies ist in der Fig. 5 dargestellt. Alternativ ist z.B. auch denkbar, dass der proximale Bereich der Betätigungsstange 21 mit dem Betätigungsring 22 anfänglich auf dem Rest der Betätigungsstange 21 aufgeschraubt ist und zum Austragen abgeschraubt wird. Anschliessend kann ein Auspresselement 80 in Form einer hohlen Gewindespindel mit einem Aussengewinde 81 und einem Betätigungsgriff 82 vom proximalen Ende her auf die Betätigungsstange 21 aufgeschoben werden, wie dies in der Fig. 6 illustriert ist. Durch Drehung der Gewindespindel im Uhrzeigersinn tritt das Aussengewinde 81 in Eingriff mit dem Innengewinde 14 der Gewindebuchse 13. Dadurch lässt sich das Auspresselement 80 so weit in die distale Richtung in das Gehäuse 10 einschrauben, bis die Gewindespindel mit ihrem distalen Ende auf den Kolben 40 stösst und diesen in die distale Richtung nach vorne schiebt. Auf diese Weise lässt sich das Produkt 74 mit reduziertem Kraftaufwand durch den Austragnippel 62 und durch eine optional in diesen Austragnippel eingeschraubte Hohlnadel 64 hindurch austragen.

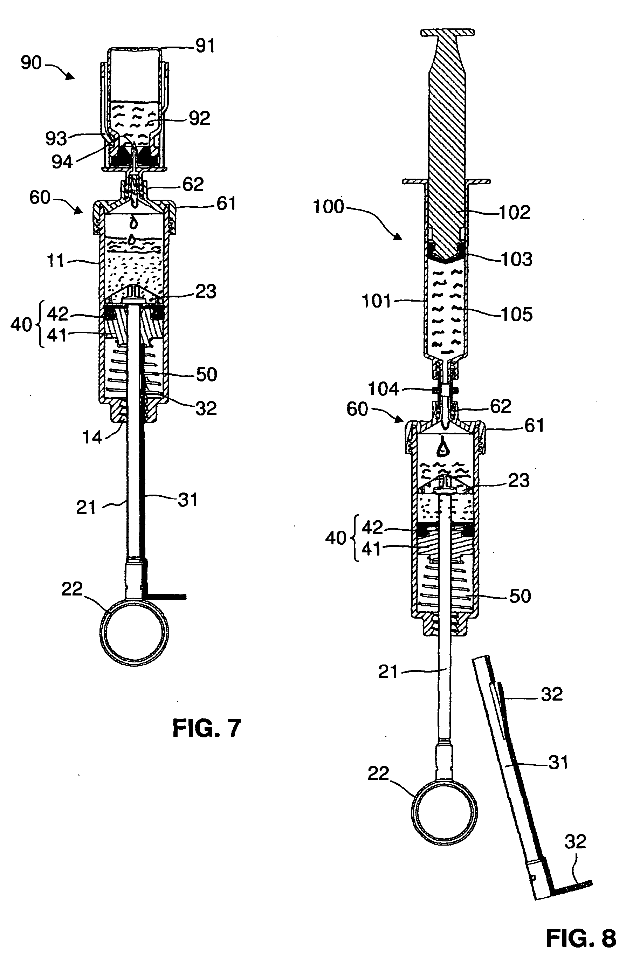

In der Fig. 7 ist eine alternative Befüllmöglichkeit für die Misch- und Austragvorrichtung der Fig. 1 illustriert. Die pulverförmige Komponente 71 wurde zuvor z. B. in der Weise, wie es in der Fig. 1 dargestellt ist, oder schon werksseitig in das Reservoir eingefüllt. In den Austragnippel 62 des Deckels 60 ist nun ein Vialhalter 90 eingeschraubt, in welchem ein handelsübliches Glasfläschchen mit Septumverschluss (ein sogenanntes Vial 91) eingesetzt ist. Dabei hält ein Rastarm 93 das Vial in einer Stellung, in der eine Hohlnadel 94 den Septumverschluss des Vials durchsticht. Auf diese Weise kann eine flüssige Komponente 92 aus dem Vial durch die Hohlnadel 94 und den Austragnippel 62 hindurch in das Reservoir im Gehäuse 10 gelangen.

Auf die Betätigungsstange 21 ist das Kopplungselement 30 aufgeschoben; die Rastklinke 32 des Kopplungselements 30 ist dabei derart eingedrückt, dass sie sich in die proximale Öffnung des Gehäuses 10 hinein erstreckt. Die Feder 50 ist zumindest teilweise entspannt. Das Mischelement 23 verschliesst die Entlüftungsöffnung 43. Um das Befüllen mit der Komponente 92 aus dem Vial 91 zu erleichtern, kann nun der Benutzer die Betätigungsstange 21 mit dem Betätigungsring 22 in die proximale Richtung gegen die Federkraft der Feder 50 nach hinten ziehen, um so im Reservoir einen Unterdruck zu erzeugen. Dadurch wird die Komponente 92 ins Reservoir gesaugt. Sobald der Benutzer die Betätigungsstange 21 so weit nach hinten gezogen hat, dass die Feder 50 vollständig komprimiert ist, verrastet die Rastklinke 32 mit dem Gehäuse 10, und der weitere Ablauf ist dann so, wie dies zuvor im Zusammenhang mit den Figuren 1 bis 6 beschrieben worden war.

Eine dritte Möglichkeit zum Befüllen des Reservoirs ist in der Fig. 8 dargestellt. Hier befindet sich ebenfalls die pulverförmige Komponente 71 schon im Reservoir. Eine flüssige Komponente 105 befindet sich im Spritzenkörper 101 einer Spritze 100. Die Spritze 100 ist über einen Adapter 104 mit dem Austragnippel 62 verbunden. Durch Druck auf eine Kolbenstange 102 kann der Benutzer den Spritzenkolben 103 in die proximale Richtung drücken, so dass die flüssige Komponente 105 in das Reservoir im Gehäuse 10 gepresst wird. Um sicherzustellen, dass dabei Gase aus dem Reservoir entweichen können, ist in diesem Fall das Kopplungselement 30 von der Betätigungsstange 21 entfernt, und das Mischelement 23 ist beabstandet vom Kolben 40 angeordnet, um die Entlüftungsöffnung 43 freizugeben.

Aus der vorstehenden Beschreibung wird ersichtlich, dass die Vorrichtung gemäss dem vorliegenden Ausführungsbeispiel auf eine Vielzahl verschiedener Weisen eingesetzt werden kann. Gleichzeitig wird ersichtlich, dass eine Vielzahl von Abwandlungen dieser Vorrichtung möglich ist, ohne den Bereich der Erfindung zu verlassen.

So kann die konkrete Ausgestaltung des Kopplungselements variieren. Weitere mögliche Ausgestaltungen von Kopplungselementen sind z. B. in der WO 2009/105905 angegeben, wobei allerdings im Zusammenhang mit der vorliegenden Erfindung bevorzugt ist, wenn das Kopplungselement von dem Betätigungselement vollständig entfernbar ist. Weitere Varianten sind auch in der schon erwähnten Patentanmeldung PCT/CH 2011/000135 vom 7. Juni 2011 angegeben.

Es können aber auch ganz andere Arten von Betätigungselementen und Kopplungselementen eingesetzt werden, als sie im vorstehenden Ausführungsbeispiel beschrieben sind. Beispielsweise ist es denkbar, die Kopplung zwischen dem Betätigungselement und dem Kolben so auszugestalten, wie dies in der EP 1 920 738 beschrieben ist. Eine Vielzahl weiterer Möglichkeiten, ein Betätigungselement für ein Mischelement mit einem Kolben zu koppeln, sind denkbar.

Falls ein Kopplungselement wie im vorliegenden Ausführungsbeispiel eingesetzt wird, kann eine andere Feststelleinrichtung als die Rastklinke 32 vorgesehen sein, um das Kopplungselement gehäusefest zu fixieren. So kann die einstückig mit dem Kopplungselement ausgebildete Rastklinke 32 auch z.B. durch eine andere Art von Rastelement, z.B. durch eine separat ausgebildete Metallfeder ersetzt werden, die in einem entsprechenden Federhalter des Kopplungselements gehalten ist. Auch ist es z. B. denkbar, auf der Aussenseite des Kopplungselements eine einfache Nase vorzusehen, welche in einer ersten Orientierung des Kopplungselements am Gehäuse 10 oder einem gehäusefesten Element angreift, um das Kopplungselement bezüglich einer Bewegung in die distale Richtung gegenüber dem Gehäuse zu fixieren. Am Gehäuse oder gehäusefesten Element kann dann eine Nut oder eine andere Struktur vorgesehen sein, welche in einer zweiten Orientierung des Kopplungselements eine Bewegung der Nase in die distale Richtung erlaubt. Die Freigabe des Kolbens relativ zum Gehäuse erfolgt dann also, indem das Kopplungselement um die Längsachse relativ zum Gehäuse verdreht wird. Alternativ ist es auch denkbar, ein Rastelement in Form z.B. einer Rastklinke öder Metallfeder nicht am Kopplungselement, sondern am Gehäuse vorzusehen, wobei dieses gehäusefest angeordnete Rastelement dann mit einer entsprechenden Gegenstruktur (z.B. einer Vertiefung oder Ausnehmung) am Kopplungselement zusammenwirkt. Dies könnte z.B. auf die in der US 2,474,496, US 2,875,761 oder in der Anmeldung PCT/CH 2010/000300 vom 25.11.2010 dargestellte Art erfolgen. Eine Vielzahl anderer Feststelleinrichtungen für das Kopplungselement sind denkbar.

Eine Kopplung zwischen dem Betätigungselement und dem Gehäuse kann aber auch völlig anders als im vorstehenden Ausführungsbeispiel hergestellt werden. So ist es z.B. denkbar, eine lösbare Verbindung direkt zwischen dem Betätigungselement und dem Gehäuse bzw. einem gehäusefesten Element vorzusehen, z.B. in Form eines in das Betätigungselement einschiebbaren Stifts, einer Rastverbindung, eines aufschiebbaren Clips usw.

Ein Kopplungselement, das das Betätigungselement und das Gehäuse relativ zueinander fixiert, kann aber auch ganz entfallen. Der Kolben kann dann mit einer völlig anderen Art von Fixiereinrichtung relativ zum Gehäuse fixiert werden. So ist es im einfachsten Fall denkbar, den Kolben mit einem seitlich durch die Gehäusewand ragenden Stift, der in eine seitliche Halteöffnung des Kolbens ragt, lösbar am Gehäuse zu fixieren.

Während im vorliegenden Beispiel die Entlüftungsöffnung 43 zwischen dem Kolben 40 und der Betätigungsstange 21 ausgebildet ist, wie dies grundsätzlich auch in der Anmeldung PCT/CH 2011/000135 beschrieben ist, kann die Entlüftungsöffnung auch an einer anderen Stelle ausgebildet sein, z. B. am distalen Ende des Gehäuses 10. Um den Austritt von Feststoffen und/oder Flüssigkeiten zu verhindern, kann in die Entlüftungsöffnung ein geeignetes Filterelement eingesetzt sein. Die Entlüftungsöffnung kann manuell durch ein Ventil verschliessbar sein, um gegebenenfalls gezielt einem Unter- oder Überdruck im Reservoir zu erzielen. Die Entlüftungsöffnung kann gegebenenfalls mit einem Anschluss versehen sein, um ein Gas zur Sterilisation, z.B. Ehtylenoxid, ins Reservoir zu leiten.

Während im vorliegenden Ausführungsbeispiel die Gewindebuchse 13 einstückig mit dem restlichen Gehäuse 10 ausgebildet ist, kann die Gewindebuchse 13 auch an einem separaten Bauteil ausgebildet sein, das z. B. vom proximalen Ende her auf das Gehäuse 10 aufsteckbar und mit diesem verrastbar oder verschraubbar sein kann. Derartige Elemente sind grundsätzlich aus dem Stand der Technik bekannt. Auch das distale Ende des Gehäuses 10 kann auf andere Weise als hier dargestellt ausgestaltet sein. Insbesondere wenn das Reservoir mit einer Komponente vorbefüllt ist, kann z. B. der Deckel 60 einstückig mit dem Gehäuse 10 ausgebildet sein.

Statt das Produkt mit einem Auspresselement in Form einer Gewindespindel auszutragen, ist es auch denkbar, die Vorrichtung so auszugestalten, dass sie z. B. in einen geeigneten Pistolendispenser einsetzbar ist. Eine Gewindebuchse kann dann ganz entfallen. Auch in dieser Hinsicht besteht eine Vielzahl weiterer Variationsmöglichkeiten.

Auch das Federelement 50 kann auf eine andere Weise als im vorliegenden Ausführungsbeispiel ausgebildet sein. So kann es sich z. B. statt einer sich spiralförmig verjüngenden Schraubenfeder auch um eine zylindrische Schraubenfeder handeln. Auch der Einsatz von Blattfedern oder anderen elastischen Elementen, z. B. auf der Basis von elastischen Kunststoffen oder Schaumstoffen, ist denkbar.

**BEZUGSZEICHENLISTE**

| | | | |
|---|---|---|---|
| 10 | Gehäuse | 62 | Austragnippel |
| 11 | Seitenwand | 63 | Verschluss |
| 12 | proximale Stirnwand | 64 | Hohlnadel |
| 13 | Gewindebuchse | 70 | Behälter |
| 14 | Innengewinde | 71 | pulverförmige Komponente |
| 15 | Aussengewinde | 72 | Behälter |
| 16 | Trichter | 73 | flüssige Komponente |
| 20 | Mischeinrichtung | 74 | Produkt |
| 21 | Betätigungsstange | 80 | Auspresselement |
| 22 | Betätigungsring | 81 | Aussengewinde |
| 23 | Mischelement | 82 | Betätigungsgriff |
| 30 | Kopplungselement | 90 | Vialhalter |
| 31 | Hülse | 91 | Vial |
| 32 | Rastklinke | 92 | flüssige Komponente |
| 33 | Grifflasche | 93 | Rastarm |
| 34 | distales Ende | 94 | Hohlnadel |
| 40 | Kolben | 100 | Spritze |
| 41 | Träger | 101 | Spritzenkörper |
| 42 | Dichtelement | 102 | Kolbenstange |
| 43 | Entlüftungsöffnung | 103 | Spritzenkolben |
| 50 | Feder | 104 | Adapter |
| 60 | Deckel | 105 | flüssige Komponente |
| 61 | Schürze | | |

## Patentansprüche

1. Vorrichtung zum Mischen und Austragen eines Produkts, aufweisend:
ein Gehäuse (10), welches ein Reservoir für das Produkt begrenzt;
ein Mischelement (23), welches im Reservoir bewegbar ist, um ein darin aufgenommenes Produkt zu durchmischen;
einen Kolben (40), welcher das Reservoir zu einem proximalen Ende hin begrenzt und welcher relativ zum Gehäuse (10) in eine distale Richtung verschiebbar ist, um das Produkt aus dem Reservoir auszutragen; und
ein Federelement (50), das dazu ausgebildet ist, den Kolben (40) gegenüber dem Gehäuse (10) in die distale Richtung mit einer Federkraft zu belasten,
**dadurch gekennzeichnet, dass**
die Vorrichtung mindestens eine Entlüftungsöffnung (43) aufweist, um den Austritt von Gas aus dem Reservoir zu ermöglichen.

2. Vorrichtung nach Anspruch 1, welche eine Fixiereinrichtung aufweist, um den Kolben (40) relativ zum Gehäuse (10) in einer Haltestellung, in der das Federelement (50) vorgespannt ist, zu fixieren.

3. Vorrichtung nach Anspruch 1 oder 2, wobei die Entlüftungsöffnung (43) derart ausgebildet ist, dass sie einen Austritt von Feststoffen verhindert, während sie einen Austritt von Gasen erlaubt.

4. Vorrichtung nach einem der vorhergehenden Ansprüche, wobei die Entlüftungsöffnung (43) am Kolben ausgebildet ist.

5. Vorrichtung nach Anspruch 4, wobei die Entlüftungsöffnung (43) durch das Mischelement (23) und/oder ein damit verbundenes Betätigungselement (21) verschliessbar ist.

6. Vorrichtung nach einem der vorhergehenden Ansprüche, wobei das Mischelement (23) mit einem Betätigungselement (21) verbunden ist, welches den Kolben (40) durchsetzt und in proximaler Richtung aus dem Reservoir herausragt, um das Mischelement (23) im Reservoir zu bewegen.

7. Vorrichtung nach Anspruch 6, welche ausserdem ein Kopplungselement (30) umfasst, welches in einen Zustand bringbar ist, in dem es das Betätigungselement (21) mindestens bezüglich einer distalen Richtung relativ zum Gehäuse (10) fixiert, so dass das Betätigungselement (21) und/oder das damit verbundene Mischelement (23) den Kolben (40) in einer Haltestellung halten, in der das Federelement (50) vorgespannt ist.

8. Vorrichtung nach Anspruch 7, wobei das Kopplungselement (30) ausserdem dazu ausgebildet ist, den Kolben (40) im Reservoir zu bewegen, so lange sich das Kopplungselement (30) in einem Zustand befindet, in dem es das Betätigungselement (21) nicht relativ zum Gehäuse fixiert.

9. Vorrichtung nach Anspruch 7 oder 8, wobei das Kopplungselement (30) derart proximal vom Kolben (40) am Betätigungselement (21) anbringbar ist, dass der Kolben (40) zwischen dem Kopplungselement (30) und dem Mischelement (23) gehalten ist.

10. Vorrichtung nach einem der Ansprüche 7-9, wobei das Betätigungselement (21) eine Betätigungsstange bildet, und wobei das Kopplungselement (30) eine lateral offene hohle Kolbenstange bildet, die in einer lateralen Richtung über die Betätigungsstange bewegbar und derart an der Betätigungsstange fixierbar ist, dass sie am Kolben (40) anliegt.

11. Vorrichtung nach einem der Ansprüche 7-10, welche eine lösbare Feststelleinrichtung (32) aufweist, die in der Haltestellung das Kopplungselement (30) relativ zum Gehäuse (10) derart fixiert, dass die Feststelleinrichtung (32) eine Bewegung des Kopplungselements (30) zumindest in die distale Richtung blockiert, während die Feststelleinrichtung (32) in einer Freigabestellung eine Bewegung des Kopplungselements (30) gemeinsam mit dem Betätigungselement (21) in die distale Richtung erlaubt.

12. Vorrichtung nach Anspruch 11, wobei die Feststelleinrichtung (32) eine Rastklinke umfasst, die derart am Kopplungselement (30) ausgebildet ist, dass sie in der Haltestellung am Gehäuse (10) angreift, um eine Bewegung des Kopplungselement (30) in die distale Richtung zu verhindern.

13. Vorrichtung nach einem der Ansprüche 6-12, wobei das Betätigungselement (21) eine Trennstelle aufweist, um einen proximalen Teil des Betätigungselements (21) zu entfernen.

14. Vorrichtung nach einem der Ansprüche 6-13, welche ausserdem ein vorzugsweise auf zumindest einen Teil des Betätigungselements (21) aufschiebbares Auspresselement (80) aufweist, um eine Auspresskraft in distaler Richtung auf den Kolben (40) auszuüben, wobei das Auspresselement (80) dazu ausgebildet ist, eine Gewindeverbindung mit einem gehäusefesten Gegenstück (13) einzugehen.

15. Vorrichtung nach Anspruch 14, wobei das gehäusefeste Gegenstück (13) einstückig mit dem Gehäuse (10) ausgebildet und in einem proximal vom Kolben (40) gelegenen Bereich des Gehäuses (10) angeordnet ist.

## Claims

1. A device for the mixing and discharging of a product, comprising:
a housing (10) which delimits a reservoir for the product;
a mixing element (23) which is movable in the reservoir in order to mix a product accommodated therein;
a piston (40) which delimits the reservoir to a proximal end and is displaceable into a distal direction relative to the housing (10) in order to discharge the product from the reservoir; and
a spring element (50) which is adapted to load the piston (40) relative to the housing (10) in the distal direction with a spring force,
**characterized in that** the device comprises at least one ventilation opening (43) in order to allow gases to escape from the reservoir.

2. The device according to claim 1, comprising a fixing device in order to fix the piston (40) relative to the housing (10) in a holding position in which the spring element (50) is pre-tensioned.

3. The device according to claim 1 or 2, wherein the ventilation opening (43) is designed in such a manner that it prevents any escape of solids, while allowing gases to escape.

4. The device according to any one of the preceding claims, wherein the ventilation opening (43) is arranged on the piston.

5. The device according to claim 4, wherein the ventilation opening (43) can be closed by the mixing element (23) and/or by an actuating element (21) connected thereto.

6. The device according to any one of the preceding claims, wherein the mixing element (23) is connected to an actuating element (21) which penetrates the piston (40) and in the proximal direction projects from the reservoir in order to move the mixing element (23) in the reservoir.

7. The device according to claim 6, further comprising a coupling element (30) which can be brought to a state in which it fixes the actuating element (21) in terms of at least a distal direction relative to the housing (10) so that the actuating element (21) and/or the interconnected mixing element (23) hold/holds the piston (40) in a holding position in which the spring element (50) is pre-tensioned.

8. The device according to claim 7, wherein the coupling element (30) is furthermore designed to move the piston (40) in the reservoir, provided the coupling element (30) is in a state in which it does not fix the actuating element (21) relative to the housing.

9. The device according to claim 7 or 8, wherein the coupling element (30) can be attached, proximally from the piston (40), to the actuating element (21) in such a manner that the piston (40) is held between the coupling element (30) and the mixing element (23).

10. The device according to any one of claims 7-9, wherein the actuating element (21) forms an actuating rod, and wherein the coupling element (30) forms a laterally-open hollow piston rod which in a lateral direction can be moved by way of the actuating rod and can be fixed to the actuating rod in such a manner that it rests against the piston (40).

11. The device according to any one of claims 7-10, comprising a detachable locking device (32) which in the holding position fixes the coupling element (30) relative to the housing (10) in such a manner that the locking device (32) blocks movement of the coupling element (30) at least in the distal direction, while the locking device (32) in a release position allows movement of the coupling element (30) together with the actuating element (21) in the distal direction.

12. The device according to claim 11, wherein the locking device (32) comprises a locking catch designed on the coupling element (30) in such a manner that in the holding position it engages the housing (10) in order to prevent any movement of the coupling element (30) in the distal direction.

13. The device according to any one of claims 6-12, wherein the actuating element (21) comprises a joint in order to remove a proximal part of the actuating element (21).

14. The device according to any one of claims 6-13, further comprising a press-out element (80), preferably slidable onto at least part of the actuating element (21), in order to exert a press-out force on the piston (40) into the distal direction, wherein the press-out element (80) is designed to establish a threaded connection with a counterpart (13) fixed to the housing.

15. The device according to claim 14, wherein the counterpart (13) which is fixed to the housing is designed in one piece with the housing (10) and is arranged in a region of the housing (10) which is situated proximally to the piston (40).

## Revendications

1. Un dispositif pour mélanger et décharger un produit, comprenant :
un boîtier (10), qui limite un réservoir pour le produit ;
un élément de mélange (23), qui est mobile dans le réservoir, pour mélanger un produit accommodé dans celui-ci ;
un piston (40), qui limite le réservoir vers une extrémité proximale et qui est mobile relativement par rapport au boîtier (10) dans une direction distale, pour décharger le produit du réservoir ; et
un élément de ressort (50), qui est adapté pour charger le piston (40) par rapport au boîtier (10) avec une force de ressort dans une direction distale,
**caractérisé en ce que**
le dispositif comprend au moins une ouverture d'évacuation (43), pour permettre l'échappement de gaz du réservoir.

2. Le dispositif selon la revendication 1, qui comprend un dispositif de fixation, pour fixer le piston (40) relativement par rapport au boîtier (10) dans une position d'arrêt, dans laquelle l'élément de ressort (50) est précontraint.

3. Le dispositif selon les revendications 1 ou 2, ou l'ouverture d'évacuation (43) est formée d'une telle manière qu'elle empêche la sortie des matériaux solides, pendant qu'elle permet la sortie des gaz.

4. Le dispositif selon l'une des revendications précédentes, où l'ouverture d'évacuation (43) est formée dans le piston.

5. Le dispositif selon la revendication 4, où l'ouverture d'évacuation (43) peut être fermée par l'élément de mélange (23) et/ou par un élément d'actionnement (21) qui est connecté avec celui-ci.

6. Le dispositif selon l'une des revendications précédentes, où l'élément de mélange (23) est connecté avec un élément d'actionnement (21), qui passe à travers le piston (40) et qui saillie du réservoir dans une direction proximale, pour déplacer l'élément de mélange (23) dans le réservoir.

7. Le dispositif selon la revendication 6, qui comprend en outre un élément de couplage (30), qui peut être mis dans un état, dans lequel il fixe l'élément d'actionnement (21) au moins par rapport à une direction distale relative au boîtier (10), d'une telle manière que l'élément d'actionnement (21) et/ou l'élément de mélange (23) qui est connecté avec celui-ci, maintiennent le piston (40) dans une position d'arrêt, dans laquelle l'élément de ressort (50) est précontraint.

8. Le dispositif selon la revendication 7, où l'élément de couplage (30) est formé en outre d'une telle manière pour déplacer le piston (40) dans le réservoir, pendant que l'élément de couplage (30) se trouve dans un état, dans lequel il ne fixe pas l'élément d'actionnement (21) relativement par rapport au boîtier.

9. Le dispositif selon la revendication 7 ou 8, où l'élément de couplage (30) peut être arrangé de manière proximale au piston (40) à l'élément d'actionnement (21) d'une telle manière que le piston (40) est maintenu entre l'élément de couplage (30) et l'élément de mélange (23).

10. Le dispositif selon l'une des revendications 7 à 9, où l'élément d'actionnement (21) forme une tige d'actionnement, et où l'élément de couplage (30) forme une tige de piston creuse latéralement ouverte, qui est déplaçable dans une direction latérale par l'intermédiaire de la tige d'actionnement et qui peut être fixée à la tige d'actionnement d'une telle manière, qu'elle repose contre le piston (40).

11. Le dispositif selon l'une des revendications 7 à 10, qui comprend un dispositif de fixation (32), qui fixe l'élément de couplage (30) relativement par rapport au boîtier (10) d'une telle manière, que le dispositif de fixation (32) bloque un mouvement de l'élément de couplage (30) au moins dans la direction distale, pendant que le dispositif de fixation (32), dans une position de libération, permet un mouvement de l'élément de couplage (30) ensemble avec l'élément d'actionnement (21) dans la direction distale.

12. Le dispositif selon la revendication 11, où le dispositif de fixation (32) comprend un cliquet d'arrêt, qui est formé d'une telle manière à l'élément de couplage (30), qu'il engage dans le boîtier (10) dans la position d'arrêt, pour empêcher un mouvement de l'élément de couplage (30) dans la direction distale.

13. Le dispositif selon l'une des revendications 6 à 12, où l'élément d'actionnement (21) comprend un endroit de séparation, pour enlever une partie proximale de l'élément d'actionnement (21).

14. Le dispositif selon l'une des revendications 6 à 13, qui comprend en outre un élément de compression (80), qui est de préférence coulissant sur au moins une partie de l'élément d'actionnement (21), pour exercer une force de compression sur le piston (40) dans la direction distale, où l'élément de compression (80) est configuré pour entrer dans une liaison filetée avec un homologue (13) fixé au boîtier.

15. Le dispositif selon la revendication 14, où l'homologue (13) fixé au boîtier est formé intégralement avec le boîtier (10) et est arrangé dans le boîtier (10) dans une région proximale du piston (40).
